(19) **Europäisches Patentamt**
**European Patent Office**
**Office européen des brevets**

(11) **EP 4 563 009 A1**

(12) **EUROPEAN PATENT APPLICATION**
published in accordance with Art. 153(4) EPC

(43) Date of publication:
**04.06.2025 Bulletin 2025/23**

(21) Application number: **23846494.5**

(22) Date of filing: **25.07.2023**

(51) International Patent Classification (IPC):
**A23L 31/15** (2016.01)    **C12N 1/20** (2006.01)
**C12N 9/54** (2006.01)    **C12N 9/56** (2006.01)
**C12N 9/58** (2006.01)

(52) Cooperative Patent Classification (CPC):
**A23L 31/15; C12N 1/20; C12N 9/54; C12N 9/58**

(86) International application number:
**PCT/JP2023/027110**

(87) International publication number:
**WO 2024/024764 (01.02.2024 Gazette 2024/05)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **25.07.2022 JP 2022118057**

(71) Applicants:
• **Amano Enzyme Inc.**
**Nagoya-shi**
**Aichi 460-8630 (JP)**

• **Amano Enzyme U.S.A. Co., Ltd.**
**Elgin, Illinois 60124 (US)**

(72) Inventor: **OKUDA, Keita**
**Kakamigahara-shi, Gifu 509-0109 (JP)**

(74) Representative: **Müller-Boré & Partner**
**Patentanwälte PartG mbB**
**Friedenheimer Brücke 21**
**80639 München (DE)**

(54) **YEAST EXTRACT PRODUCTION METHOD**

(57)    The purpose of the present invention is to provide a method for producing a yeast extract having a high peptide ratio. This yeast extract production method includes a step for causing a protease and/or a glutaminase to act on yeast, said protease being selected from the group consisting of proteases derived from the genus Bacillus, proteases derived from the genus Geobacillus, and proteases derived from the genus Rhizopus, and makes it possible to produce a yeast extract having a high peptide ratio.

**EP 4 563 009 A1**

**Description**

Technical Field

The present invention relates to a yeast extract production method, and more specifically to a yeast extract production method providing a high peptide ratio or capable of promoting the production of lactic acid bacteria in addition to the high peptide ratio.

Background Art

Yeast extracts are blended in various food products and the like for the purpose of applying or enhancing a preferred taste such as an umami taste or a rich taste, blended in a culture medium for the purpose of being used as a nitrogen nutrient source at the time of microorganism culture, and applied to a wide range of applications. Furthermore, the demand for the yeast extracts is on an increasing trend against the background of a recent increase in natural consciousness, health consciousness, and the like. Therefore, development for the purpose of improving the added value of the yeast extract has been conducted.

For example, in order to improve the content of the taste component of the yeast extract, a method is known in which a yeast suspension is subjected to alkaline extraction, and then a protease and a ribonuclease in cells are heat-inactivated, and then 5'-phosphodiesterase and 5'-adenylate deaminase are reacted to obtain a yeast extract containing 8% or more of each of 5'-guanylic acid and 5'-inosinic acid, which are tasty 5'-nucleotides, and 16% or more of a combination of a peptide and a free amino acid (PTL 1).

In order to improve the sustainment of the exercise capacity, the effect of preventing/recovering fatigue, and the effect on chronic fatigue syndrome of the yeast extract, a yeast extract-containing composition is known in which a yeast extract containing a predetermined amount of vitamin B1, a yeast extract containing a predetermined amount of peptide, and a yeast extract containing a predetermined amount of glutathione are combined at a predetermined ratio (PTL 2).

Citation List

Patent Literature

PTL 1: Japanese Patent Laid-open Publication No. 06-113789
PTL 2: Japanese Patent Laid-open Publication No. 2009-107962

Summary of Invention

Technical Problem

The peptide contained in the yeast extract is considered to be a particularly important element in the application of the added value of the yeast extract in that the peptide includes a functional peptide that exerts a useful function in the body by ingestion in addition to applying a preferable taste to a food product. However, in a yeast extract production method known so far, a peptide ratio in the obtained yeast extract is not yet sufficient.

The purpose of the present invention is to provide a method for producing a yeast extract having a high peptide ratio.

Solution to Problem

The present inventor has found that a yeast extract having a high peptide ratio can be obtained by causing a protease and/or a glutaminase to act on yeast, the protease being selected from the group consisting of a protease derived from the genus Bacillus, a protease derived from the genus Geobacillus, and a protease derived from the genus Rhizopus. The present inventor has also found that when lactic acid bacteria are cultured using the yeast extract thus obtained, an excellent ability to promote the production of lactic acid bacteria is exhibited. The present invention has been completed by further conducting studies based on such findings.

That is, the present invention provides the inventions of the following aspects.

Item 1. A yeast extract production method including the step of causing a protease and/or a glutaminase to act on yeast, the protease being selected from the group consisting of a protease derived from the genus Bacillus, a protease derived from the genus Geobacillus, and a protease derived from the genus Rhizopus.

Item 2. The production method according to Item 1, in which the protease derived from the genus Bacillus is a protease derived from Bacillus licheniformis.

Item 3. The production method according to Item 1, in which the protease derived from the genus Geobacillus is a protease derived from Geobacillus stearothermophilus (also including a strain referred to as Bacillus stearothermophilus in the past).

Item 4. The production method according to any one of Items 1 to 3, in which the protease derived from the genus Rhizopus is a protease derived from Rhizopus niveus.

Item 5. The production method according to any one of Items 1 to 4, in which the step is carried out under the condition of pH 7 to 9 using at least the protease derived from the genus Bacillus and/or the protease derived from the genus Geobacillus.

Item 6. The production method according to any one of Items 1 to 4, in which the step is carried out under the condition of pH 3 to 6 using at least the protease derived from the genus Rhizopus.

Item 7. A yeast extract obtained by the production method according to any one of Items 1 to 6.

Item 8. An enhancer for a peptide ratio of a yeast extract, including a protease and/or a glutaminase, the protease being selected from the group consisting of a protease derived from the genus Bacillus, a protease derived from the genus Geobacillus, and a protease derived from the genus Rhizopus.

Item 9. An enhancer for an ability to produce lactic acid bacteria of a yeast extract, including a protease and/or a glutaminase, the protease being selected from the group consisting of a protease derived from the genus Bacillus, a protease derived from the genus Geobacillus, and a protease derived from the genus Rhizopus.

Item 10. A method for producing a culture of lactic acid bacteria, including the step of culturing lactic acid bacteria using a medium containing a yeast extract obtained by the production method according to any one of Items 1 to 6.

Advantageous Effects of Invention

According to the present invention, a yeast extract having a high peptide ratio is provided. According to the present invention, there is also provided a yeast extract having an excellent ability to promote the production of lactic acid bacteria when used in the cultivation of the lactic acid bacteria.

Description of Embodiments

1. Yeast extract production method

A yeast extract production method according to the present invention includes the step of causing a protease and/or a glutaminase (hereinafter, also described as an enzyme) to act on yeast, the protease being selected from the group consisting of a protease derived from the genus Bacillus, a protease derived from the genus Geobacillus, and a protease derived from the genus Rhizopus. In the step, more specifically, a mixed solution in which yeast and a predetermined enzyme are typically contained in water is subjected to conditions under which the predetermined enzyme acts. This makes it possible to obtain a yeast extract having a high peptide ratio. Also, this makes it possible to obtain a yeast extract having an excellent ability to promote the production of lactic acid bacteria when used in the cultivation of the lactic acid bacteria.

1-1. Yeast

The yeast used in the present invention is not particularly limited, and typical examples include yeast suitable for use in a food product. Specific examples thereof include yeast of the genus Saccharomyces such as Saccharomyces cerevisiae and Saccharomyces pastorianus; yeast of the genus Candida such as Candida utilis; yeast of the genus Kluyveromyces such as Kluyveromyces lactis and Kluyveromyces marxianus; yeast of the genus Pichia such as Pichia pastoris; yeast of the genus Debaryomyces such as Debaryomyces hansenii; yeast of the genus Zygosaccharomyces such as Zygosaccharomyces mellis; and yeast (baker's yeast, beer's yeast, yeast recovered after brewing, and the like) used or produced in the food industry. These yeasts may be used singly or in combination of two or more kinds thereof.

Among these yeasts, yeast of the genus Saccharomyces is preferred, and Saccharomyces cerevisiae is more preferred, from the viewpoint of further enhancing the peptide ratio improving effect of the yeast extract and/or the ability to promote the production of lactic acid bacteria.

The form of the yeast used in the present invention is not particularly limited, and examples thereof include a slurry form (more specifically, yeast concentrate) and a dry form (more specifically, dry yeast).

The content of the yeast contained in the mixed solution is not particularly limited, and is, for example, 1 to 25 wt%, preferably 5 to 20 wt%, more preferably 7 to 15 wt%, and still more preferably 9 to 12 wt%.

It is preferable that an endogenous enzyme is deactivated in the yeast. A method for deactivating the endogenous enzyme is not particularly limited, and can be appropriately determined by those skilled in the art, and a method for heating at 85 to 95°C for 20 to 40 minutes is preferable.

1-2. Predetermined enzyme

In the present invention, a protease (hereinafter, also described as a predetermined protease) and/or a glutaminase, the protease being selected from the group consisting of a protease derived from the genus Bacillus, a protease derived from the genus Geobacillus, and a protease derived from the genus Rhizopus are used as a predetermined enzyme. The protease in the present invention refers to an endo-type peptidase.

The protease derived from the genus Bacillus is not particularly limited, and examples thereof include proteases derived from Bacillus amyloliquefaciens, Bacillus licheniformis, Bacillus cereus, Bacillus clausii, Bacillus intermedius, Bacillus lentus, Bacillus thermoproteolyticus, and the like. These proteases derived from genus Bacillus may be used singly or in combination of two or more kinds thereof.

The protease derived from the genus Geobacillus is not particularly limited, and examples thereof include a protease derived from Geobacillus stearothermophilus (also including a strain referred to as Bacillus stearothermophilus in the past).

The protease derived from the genus Rhizopus is not particularly limited, and examples thereof include proteases derived from Rhizopus niveus, Rhizopus chinensis, Rhizopus delemar, and Rhizopus oryzae. These proteases derived from genus Rhizopus may be used singly or in combination of two or more kinds thereof.

The glutaminase is not particularly limited, and examples thereof include glutaminases derived from the genus Cryptococcus, the genus Bacillus, and the genus Aspergillus. Examples of the glutaminase derived from the genus Bacillus include glutaminase derived from Bacillus amyloliquefaciens. These glutaminases may be used singly or in combination of two or more kinds thereof.

Among these predetermined enzymes, from the viewpoint of further enhancing the peptide ratio improving effect and/or the ability to promote the production of lactic acid bacteria of the yeast extract, preferred are a protease derived from the genus Bacillus, a protease derived from the genus Geobacillus, a protease derived from the genus Rhizopus, and a glutaminase derived from the genus Bacillus, more preferred are a protease derived from the genus Bacillus, a protease derived from the genus Geobacillus, a protease derived from the genus Rhizopus, and a glutaminase derived from Bacillus amyloliquefaciens, still more preferred are a protease derived from the genus Bacillus, a protease derived from the genus Geobacillus, and a protease derived from the genus Rhizopus, and yet still more preferred are a protease derived from Bacillus licheniformis, a protease derived from Geobacillus stearothermophilus (also including a strain referred to as Bacillus stearothermophilus in the past), and a protease derived from Rhizopus niveus.

In the present invention, from the viewpoint of further enhancing the peptide ratio improving effect and/or the ability to promote the production of lactic acid bacteria of the yeast extract, it is particularly preferable to combine a predetermined protease with a glutaminase, it is more preferable to combine a protease selected from the group consisting of a protease derived from Bacillus licheniformis, a protease derived from Geobacillus stearothermophilus (including a strain referred to as Bacillus stearothermophilus in the past), and a protease derived from Rhizopus niveus with a glutaminase derived from the genus Bacillus, and it is still more preferable to combine a protease selected from the group consisting of a protease derived from Bacillus licheniformis, a protease derived from Geobacillus stearothermophilus (also including a strain referred to as Bacillus stearothermophilus in the past), and a protease derived from Rhizopus niveus with a glutaminase derived from Bacillus amyloliquefaciens.

The predetermined enzyme can be prepared from a culture solution of a microorganism from which the predetermined enzyme is derived or a heterologous host into which a gene encoding the predetermined enzyme is introduced. Specific examples of the preparation method include a method for recovering a target enzyme from the above-mentioned culture solution of the microorganism or cells. For example, in the case of using a microorganism that secretes a target enzyme to the outside of the cells, the enzyme can be separated and/or purified after recovering the cells from the culture solution in advance by filtration, centrifugation, or the like as necessary. For example, in the case of using a microorganism that does not secrete a target enzyme to the outside of the cells, the enzyme can be separated and/or purified after recovering the cells from the culture solution in advance as necessary and then disrupting the cells by a pressurization treatment, an ultrasonic treatment, or the like to expose the enzyme. As an enzyme separation and/or purification method, a known protein separation and/or purification method can be used without particular limitation, and examples thereof include a centrifugal separation method, a UF concentration method, a salting-out method, and various chromatography methods using an ion exchange resin, and the like. The separated and/or purified enzyme may be powdered by a drying method such as freeze drying or reduced pressure drying, or may be powdered using an appropriate excipient and/or drying aid in the drying method. The separated and/or purified enzyme may be liquefied by adding an appropriate additive and performing filtration sterilization.

The predetermined enzyme may be prepared by the above method, or may be a commercially available product. Preferable examples of the commercially available product include proteases derived from Bacillus licheniformis, Bacillus stearothermophilus (reclassified as Geobacillus stearothermophilus), and Rhizopus niveus manufactured by Amano Enzyme Inc., and a glutaminase derived from Bacillus amyloliquefaciens manufactured by Amano Enzyme Inc.

The amount of the predetermined protease used is not particularly limited, and is, for example, 4 U or more per 1 g of the yeast, and is preferably 20 U or more, more preferably 40 U or more, still more preferably 200 U or more, still more preferably 360 U or more, 660 U or more, 760 U or more, or 860 U or more from the viewpoint of further enhancing the peptide ratio improving effect and/or the ability to promote the production of lactic acid bacteria of the yeast extract. The upper limit of the range of the amount of the predetermined protease used per 1 g of the yeast is not particularly limited, and examples thereof include 50,000 U or less, 10,000 U or less, 5000 U or less, 2000 U or less, 1500 U or less, 1000 U or less, 940 U or less, 840 U or less, 740 U or less, 540 U or less, or 440 U or less.

The amount of the glutaminase used is not particularly limited, and the amount of the glutaminase used per 1 g of the yeast is, for example, 0.01 U or more. The amount is preferably 0.05 U or more, more preferably 0.1 U or more, still more preferably 0.5 U or more, and yet still more preferably 0.9 U or more from the viewpoint of further enhancing the peptide ratio improving effect and/or the ability to promote the production of lactic acid bacteria of the yeast extract. The upper limit of the range of the amount of the glutaminase used per 1 g of the yeast is not particularly limited, and examples thereof include 50 U or less, 10 U or less, 5 U or less, 2 U or less, 1.5 U or less, or 1.1 U or less.

When the predetermined protease and the glutaminase are used in combination, the use ratio of the predetermined protease and the glutaminase is determined based on the use amount of each enzyme, and the use amount of the protease per 1 U of the glutaminase is preferably 25 U or more, more preferably 50 U or more, still more preferably 200 U or more, yet still more preferably 360 U or more, 500 U or more, or 750 U or more, and the upper limit of the range of the use amount of the protease per 1 U of the glutaminase is, for example, 50,000 U or less, preferably 10,000 U or less, more preferably 5000 U or less, still more preferably 2000 U or less, yet still more preferably 1500 U or less, 1000 U or less, or 500 U or less, from the viewpoint of further enhancing the peptide ratio improving effect and/or the ability to promote the production of lactic acid bacteria of the yeast extract.

The activity of the protease is measured with the Folin method at pH 3 (in the case of acidic protease) or pH 8 (in the case of neutral protease and alkaline protease) using casein as a substrate. That is, the activity of the protease is defined so that when an enzyme reaction is carried out at pH 3 (in the case of acidic protease) or pH 8 (in the case of neutral protease and alkaline protease) with a conventional method using casein as a substrate, the amount of an enzyme that increases a folin reagent-coloring substance by an amount equivalent to 1 μg of tyrosine per minute is 1 unit (1 U).

The activity of the glutaminase is defined so that L-glutamine as a substrate is used, and the amount of an enzyme that produces 1 μmol L-glutamic acid per minute is 1 unit (1 U).

1-3. Reaction conditions and the like

Reaction conditions (pH, temperature, and time) for subjecting the mixed solution to the action of a predetermined enzyme may be appropriately determined, preferably further through preliminary experiments, depending on the pH characteristics and temperature characteristics of the enzyme to be used, a yeast preparation scale, and the like.

For example, the pH (25°C) of the mixed solution is, for example, 3 to 11, preferably 4 to 10, more preferably 4 to 9, and still more preferably 4.5 to 8.5, 4.5 to 7, 4.5 to 6, 6 to 8.5, or 7 to 8.5.

More specifically, when a neutral protease or an alkaline protease is used as the predetermined enzyme, and preferably when a protease derived from the genus Bacillus and/or a protease derived from the genus Geobacillus are used, the pH (25°C) of the mixed solution is preferably 7 to 9, and preferably 7.5 to 8.5. When an acidic protease is used as the predetermined enzyme, the pH (25°C) of the mixed solution is preferably 3 to 6, more preferably 4 to 5.5, and still more preferably 4.5 to 5.5.

Naturally, when a yeast extract is prepared under a condition where the pH is relatively high, the peptide ratio is significantly reduced, but according to the present invention, even when an extract is prepared under a condition where the pH is relatively high, the peptide ratio can be effectively improved. From such a viewpoint, a suitable example of the pH (25°C) of the mixed solution is 7 to 9, and preferably 7.5 to 8.5.

Examples of a reaction temperature include 30 to 80°C, preferably 40 to 70°C, more preferably 40 to 60°C, and still more preferably 45 to 55°C.

A reaction time is, for example, 5 minutes to 12 hours, preferably 10 minutes to 6 hours, and more preferably 30 minutes to 3 hours.

In the present invention, when a glutaminase and a predetermined protease are used in combination, the order in which the glutaminase and the predetermined protease are caused to act on yeast is not particularly limited, and each enzyme may be caused to act sequentially in any order, or both the enzymes may be caused to act simultaneously. In the above case, the glutaminase and the predetermined protease are preferably caused to act simultaneously from the viewpoint of further enhancing the peptide ratio improving effect and/or the ability to promote the production of lactic acid bacteria of the yeast extract.

1-4. Other steps

The production method of the present invention can include any other steps in addition to the step of causing the predetermined enzyme to act. Specific examples of the other steps include: a step of inactivating an endogenous enzyme of yeast as a step performed before the above step; a step of inactivating the predetermined enzyme, a step of recovering a yeast extract (examples thereof include a method in which a supernatant is obtained as an extract by solid-liquid separation such as centrifugation) as a step performed after the above step; and a step of concentrating a yeast extract, a step of drying a yeast extract (examples thereof include freeze drying, vacuum drying, and spray drying). **In** the production method of the present invention, any one of the other steps may be included alone, or two or more thereof may be included in combination.

2. Yeast extract

The yeast extract of the present invention is a yeast extract obtained by the above "1. Method for producing yeast extract". The yeast extract of the present invention contains a peptide at a high ratio. In the present invention, the peptide refers to a peptide having a peak in a molecular weight of 400 or more and 1000 or less in molecular weight distribution chromatography. In the present invention, the ratio of the peptide in the yeast extract refers to the ratio of the weight of the peptide contained in the yeast extract to the weight of a free amino acid contained in the yeast extract ([Weight of peptide contained in yeast extract]/[weight of free amino acid contained in yeast extract]).

The peptide ratio of the yeast extract of the present invention exceeds 1. The specific peptide ratio may vary depending on pH conditions or the like at the time of the reaction, and is, for example, 1.7 or more, preferably 2 or more, more preferably 2.5 or more, still more preferably 3 or more, and yet still more preferably 3.5 or more. The upper limit of the range of the peptide ratio is not particularly limited, and is, for example, 4.5 or less or 4 or less.

Since the yeast extract of the present invention has a high peptide ratio, the yeast extract can be used for applications such as improvement in a taste, taste masking, flavoring, and seasoning of a food and drink product (including both a food and drink product for humans and a feed for animals other than humans, the same applies hereinafter); applications such as enhancement of nutrition of a food and drink product and addition of nutrition; and an application of a nutrient source blended in a culture medium for microorganisms.

The yeast extract of the present invention exhibits an excellent ability to promote the production of lactic acid bacteria when the lactic acid bacteria are cultured, in addition to having a high peptide ratio. In the yeast extract of the present invention, the level of the peptide ratio and the level of the effect of promoting the production of lactic acid bacteria do not correspond to each other, and thus specific components or compositions contributing to the promotion of the production of lactic acid bacteria of the yeast extract of the present invention are not clear, but it is considered that at least any specific component or composition constituting a specific wide variety of compound groups including the components contained in the yeast and the components generated by the action of the predetermined enzyme among the components exhibits the effect of promoting the production of lactic acid bacteria. Therefore, the yeast extract of the present invention is preferably used for the application of a nutrient source to be blended in a culture medium of lactic acid bacteria (that is, for culturing lactic acid bacteria).

The form of the yeast extract is not particularly limited, and may be appropriately determined according to the applications and the like, and examples thereof include a fluid state such as a liquid state, a slurry state, and a paste state, and a solid state, and specific examples of the solid state include a powder state, a fine granular state, and a granular state.

3. Enhancer for peptide ratio of yeast extract

The high peptide ratio of the obtained yeast extract can be enhanced by causing the above determined enzyme (a protease and/or a glutaminase, the protease being selected from the group consisting of a protease derived from the genus Bacillus, a protease derived from the genus Geobacillus, and a protease derived from the genus Rhizopus) to act on yeast. Therefore, the present invention also provides an enhancer for a peptide ratio of a yeast extract containing a protease and/or a glutaminase, the protease being selected from the group consisting of a protease derived from the genus Bacillus, a protease derived from the genus Geobacillus, and a protease derived from the genus Rhizopus.

In the enhancer for the peptide ratio of the yeast extract, the types and amounts and the like of the components used are as shown in the column of "1. Method for producing yeast extract".

4. Enhancer for ability to produce lactic acid bacteria of yeast extract

The ability to produce the lactic acid bacteria of the obtained yeast extract can be enhanced by causing the above determined enzyme (a protease and/or a glutaminase, the protease being selected from the group consisting of a protease derived from the genus Bacillus, a protease derived from the genus Geobacillus, and a protease derived from the genus Rhizopus) to act on yeast. Therefore, the present invention also provides an enhancer for an ability to produce lactic acid bacteria of a yeast extract containing a protease and/or a glutaminase, the protease being selected from the group consisting of a protease derived from the genus Bacillus, a protease derived from the genus Geobacillus, and a protease derived from the genus Rhizopus.

In the enhancer for an ability to produce lactic acid bacteria of a yeast extract, the types and amounts and the like of the components used are as shown in the above column of "1. Method for producing yeast extract".

5. Method for producing culture of lactic acid bacteria

As described above, the yeast extract shown in the above column of "2. Yeast extract" is useful for the application of the nutrient source to be blended in the culture medium of the lactic acid bacteria (that is, for culturing the lactic acid bacteria). Therefore, the present invention also provides a method for producing a culture of lactic acid bacteria including the step of culturing lactic acid bacteria using a medium containing a yeast extract obtained by the above "1. Method for producing yeast extract".

The lactic acid bacteria to be cultured are not particularly limited, and examples thereof include lactic acid bacteria of the genus Lactobacillus, the genus Bifidobacterium, and the like. Examples of the genus Lactobacillus include Lactobacillus acidophilus, Lactobacillus casei, Lactobacillus rhamnosus, and Lactobacillus gasseri. One of these lactic acid bacteria may be cultured alone, or a plurality of lactic acid bacteria may be co-cultured.

As the medium, any medium applicable to the culture of the lactic acid bacteria can be used, and specific examples thereof include an MRS medium and a GYP medium.

The content of the yeast extract in the medium is not particularly limited, and may be adjusted so as to have a protein content equivalent to that of a known yeast extract.

Culture conditions may be appropriately determined by those skilled in the art in consideration of the pH characteristics, temperature characteristics, and the like of the lactic acid bacteria.

Examples

Hereinafter, the present invention will be specifically described with reference to Examples, but the present invention is not limited to the following Examples.

[Yeasts and enzymes used]

The following yeasts and enzymes were used.

[Table 1]

| Material Name | Derivation | Manufacturer | Details |
|---|---|---|---|
| Yeast Powder | Derived from Saccharomyces cerevisiae | Red Star Yeast | Product Name: Active Dry Yeast |
| Glutaminase | Derived from Bacillus amyloliquefaciens | Amano Enzyme | - |
| Protease A | Derived from Rhizopus niveus | Amano Enzyme | Acidic Protease |
| Protease B | Derived from Bacillus amyloliquefaciens | Amano Enzyme | Neutral Protease |
| Protease C | Derived from Bacillus stearothermophilus (reclassified as Geobacillus stearothermophilus) | Amano Enzyme | Neutral Protease |
| Protease D | Derived from Bacillus licheniformis | Amano Enzyme | Alkaline protease |
| Protease E | Derived from Aspergillus oryzae | Amano Enzyme | Acidic Protease |
| Protease F | Derived from Aspergillus meleus | Amano Enzyme | Neutral Protease |
| Protease G | Derived from Aspergillus oryzae | Amano Enzyme | Neutral Protease |

[Protease activity value measurement method]

After 5 mL of a 0.6% (w/v) casein solution (0.05 mol/L sodium hydrogen phosphate, pH 8.0 [in the case of neutral proteases and alkaline proteases other than protease G], 0.05 mol/L sodium hydrogen phosphate, pH 6.0 [in the case of protease G], or 0.7% (v/w) lactic acid, pH 3.0 [in the case of acidic protease]) was warmed at 37°C for 10 minutes, 1 mL of a sample solution containing a protease was added thereto, followed by immediately shaking. After this solution was caused to stand at 37°C for 10 minutes, 5 mL of a trichloroacetic acid solution (0.44 mol/L trichloroacetic acid) was added thereto, followed by shaking. The mixture was caused to stand at 37°C for 30 minutes again, and filtered. 3 mL of a first filtrate was removed, and 2 mL of a next filtrate was weighed. Then, 5 mL of a 0.55 mol/L sodium carbonate solution and 1 mL of a folin reagent (1→3) were added to the next filtrate, followed by shaking well. The mixture was caused to stand at 37°C for 30 minutes. An absorbance AT of this solution (enzyme reaction solution) at a wavelength of 660 nm was measured using water as a control.

Separately, a solution (blank) was prepared by performing the same operation as in the enzyme reaction solution described above except that after 1 mL of a sample solution containing a protease was weighed, and 5 mL of a trichloroacetic acid solution (0.44 mol/L trichloroacetic acid) was added, followed by shaking, 5 mL of a 0.6% (w/v) casein solution (0.05 mol/L sodium hydrogen phosphate, pH 8.0 [in the case of neutral proteases and alkaline proteases other than protease G], 0.05 mol/L sodium hydrogen phosphate, pH 6.0 [in the case of protease G], or 0.7% (v/v) lactic acid, pH 3.0 [in the case of acidic protease]) was added, followed by immediately shaking. The mixture was caused to stand at 37°C for 30 minutes, and an absorbance AB of the solution was measured.

The amount of an enzyme that caused an increase in the amount of a folin reagent coloring substance corresponding to 1 μg of tyrosine per minute was defined as 1 unit (1 U).

Each of 1 mL, 2 mL, 3 mL and 4 mL of 1 mg/mL tyrosine standard stock solutions (0.2 mol/L hydrochloric acid) was weighed, and a 0.2 mol/L hydrochloric acid solution was added thereto to make 100 mL. 2 mL of each solution was weighed, 5 mL of a 0.55 mol/L sodium carbonate solution and 1 mL of a folin reagent (1→3) were added thereto, followed by immediately shaking. The mixture was caused to stand at 37°C for 30 minutes. For these solutions, absorbances A1, A2, A3, and A4 at a wavelength of 660 nm were measured using, as a control, a solution obtained by weighing 2 mL of a 0.2 mol/L hydrochloric acid solution and performing the same operation as described above. The absorbances A1, A2, A3 and A4 were plotted on the vertical axis, and the amount of tyrosine (μg) in 2 mL of each solution was plotted on the horizontal axis. A calibration curve was prepared to determine the amount of tyrosine (μg) with respect to an absorbance difference of 1.

[Expression 1]

$$\text{Protease activity (U/g, U/mL)} = (AT - AB) \times F \times 11/2 \times 1/10 \times 1/M$$

AT: Absorbance of enzyme reaction solution
AB: Absorbance of blank
F: Tyrosine amount when absorbance difference obtained from tyrosine calibration curve is 1 (μg)
11/2: Conversion coefficient to total solution amount after reaction stop
11/10: Conversion factor for reaction time of 1 minute
M: Amount of sample in 1 mL of sample solution (g or mL)

[Glutaminase activity value measuring method]

An appropriate amount of an enzyme was weighed, and an acetate buffer solution (0.01 mol/L, pH 6.0, containing polyoxyethylene (10) octylphenyl ether) was added thereto to dissolve or uniformly disperse the enzyme to make 50 mL, or this was further diluted 10 times or 100 times with the same buffer solution to prepare a sample solution.

2.0 g of L-glutamine was weighed, and 70 mL of water was added thereto to dissolve the L-glutamine. Then, 10 mL of an acetate buffer solution (1 mol/L) having a pH of 6.0 and water were added to make 100 mL, thereby obtaining a substrate solution. 1 mL of the sample solution was weighed, and heated in a water bath at 37°C for 5 minutes. 1 mL of a substrate solution that had been heated to 37°C in advance was added thereto, followed by immediately shaking. The mixture was further warmed at 37°C for 10 minutes, and 1 mL of perchloric acid (83→1000) was then added thereto, followed by shaking. The mixture was immediately cooled in ice water for 1 minute or more. However, perchloric acid having a mass fraction of 60% was used. To this solution, 1 mL of a sodium hydroxide solution (3→100) was added, followed by shaking to obtain a test solution. Separately, 1 mL of a sample solution was weighed, and 1 mL of perchloric acid (83→1000) was added thereto, followed by shaking. The mixture was heated in a water bath at 37°C for 5 minutes, and 1 mL of a substrate solution was then added thereto, followed by shaking. The mixture was cooled in ice water for 1 minute or more. To this solution, 1 mL of a sodium hydroxide solution (3→100) was added, followed by shaking to obtain a comparative solution. When an absorbance at a wavelength of 555 nm is measured using an L-glutamic acid measuring kit (manufactured by YAMASA CORPORATION), the absorbance of the solution obtained by adding the test solution is larger than the absorbance of the solution obtained by adding the comparative solution. When there was turbidity in the test solution and the comparative solution of which the absorbances are measured, centrifugation was performed, and a supernatant solution was measured. The amount of an enzyme producing 1 μmol L-glutamic acid per minute was defined as 1 unit (1 U).

Glutaminase activity (U/g, U/mL) = (AS - ASB)·(AR - ARB) $\times$ 250 $\times$ 4 $\times$ 1/147 $\times$ 1/10 $\times$ n　　　　　　[Expression 2]

    As: Absorbance of reaction solution
    ASB: Absorbance of blank solution
    AR: Absorbance of L-glutamic acid standard solution (250 mg/L)
    ARB: Absorbance of water blank solution
    250: Concentration of L-glutamic acid standard solution (mg/L)
    4: Total solution amount of reaction
    147: Molecular weight of L-glutamic acid
    10: Reaction time (minute)
    n: Dilution multiple of sample

[Test Example 1]

A yeast powder was suspended in purified water to prepare a 10% (w/w) yeast suspension, and the yeast suspension was treated at 90°C for 30 minutes to deactivate an endogenous enzyme. After cooling, the pH was adjusted to 5 or 8 with 1 N hydrochloric acid or 1 N sodium hydroxide. The obtained yeast solution was dispensed into 50 g aliquots, followed by preincubating at 50°C and 200 rpm for 15 minutes. Then, enzymes in amounts shown in Tables 2 and 3 were added, followed by further reacting for 2 hours. After the reaction, the enzyme was deactivated by a treatment at 90°C for 15 minutes, and centrifugation (2000 x g, 30 min) was performed. The supernatant was collected as a yeast extract.
The peptide ratio of the obtained yeast extract was calculated based on the following formula. The results are shown in Tables 2 and 3.

[Expression 3]

$$\text{peptide} = \frac{\text{peptide content (mg/mL)}}{\text{amino\quad acid\quad content}}$$

An "amino acid content" in the above formula refers to the content of a free amino acid, and was quantified using an L-amino acid quantification kit (Sigma-Aldrich). A "peptide content" in the above formula was calculated based on the following formula.

[Expression 4]

$$\text{Peptide content (mg/mL)} = \text{protein content (mg/mL)} \times \text{peptide area ratio in HPLC}$$

A "protein content" in the above formula was quantified using a total protein quantification kit (Sigma-Aldrich) based on the Lowry method. A "peptide area ratio in HPLC" in the above formula was determined by subjecting the obtained yeast extract to an HPLC column in which TSKgel G2000SWxl and YMC-Pack Diol-60 were **connected** (As a separation solvent, 0.1 M potassium phosphate buffer solution (pH 7) + 0.2 M sodium chloride/acetonitrile = 70/30 (volume ratio) was used, a flow rate was 0.35 mL/min, and a detection wavelength was 205 nm.) to obtain a chromatography chart showing a molecular weight distribution, and determining a ratio of an area occupied by a peptide peak having a molecular weight (calculated from a calibration curve obtained from an HPLC peptide mixture standard product (Sigma-Aldrich)) of 400 or more and 1000 or less to a total area occupied by a peak having a molecular weight of 80 or more and 60,000 or less.
Furthermore, for the enzyme extract having a peptide ratio exceeding 1, the relative value of the peptide ratio when the peptide ratio in the enzyme extract prepared in the same manner except that the enzyme was not used was defined as 1 was determined as a peptide ratio improvement index. The results are shown in Tables 2 and 3.

[Table 2]

| | | Comparative Example 1 | Example 1 | Example 2 | Example 3 | Example 4 | Comparative Example 2 | Comparative Example 3 | Comparative Example 4 |
|---|---|---|---|---|---|---|---|---|---|
| Glutaminase | Derived from Bacillus amyloliquefaciens | - | 1 | - | - | 1 | - | - | - |
| Protease A | Derived from Rhizopus niveus | - | - | 400 | - | 400 | - | - | - |
| Protease B | Derived from Bacillus amyloliquefaciens | - | - | - | 700 | - | - | - | - |
| Protease C | Derived from Bacillus stearothermophilus † | - | - | - | - | - | - | - | - |
| Protease D | Derived from Bacillus licheniformis | - | - | - | - | - | - | - | - |
| Protease E | Derived from Aspergillus oryzae | - | - | - | - | - | 400 | - | - |
| Protease F | Derived from Aspergillus meleus | - | - | - | - | - | - | 6000 | - |
| Protease G | Derived from Aspergillus oryzae | - | - | - | - | - | - | - | 500 |
| Treatment pH | | 5 | 5 | 5 | 5 | 5 | 5 | 5 | 5 |
| Peptide Ratio | | 1.4 | 1.8 | 2.6 | 1.8 | 2.8 | 1.5 | 1.5 | 1.5 |
| Peptide Ratio Improvement Index | | (Reference) | 1.31 | 1.86 | 1.26 | 2.02 | 1.07 | 1.07 | 1.07 |

In Table, a numerical value indicating the amount of each enzyme used is represented by an activity value per 1 g of yeast.
† Bacillus stearothermophilus is reclassified as Geobacillus stearothermophilus.

[Table 3]

| | | Comparative Example 5 | Example 5 | Example 6 | Example 7 | Example 8 | Comparative Example 6 |
|---|---|---|---|---|---|---|---|
| Glutaminase | Derived from Bacillus amyloliquefaciens | - | 1 | - | - | 1 | - |
| Protease A | Derived from Rhizopus niveus | - | - | - | - | - | - |
| Protease B | Derived from Bacillus amyloliquefaciens | - | - | - | - | - | - |
| Protease C | Derived from Bacillus stearothermophilus † | - | - | 900 | - | - | - |
| Protease D | Derived from Bacillus licheniformis | - | - | - | 800 | 800 | - |
| Protease E | Derived from Aspergillus oryzae | - | - | - | - | - | - |
| Protease F | Derived from Aspergillus meleus | - | - | - | - | - | 6000 |
| Protease G | Derived from Aspergillus oryzae | - | - | - | - | - | - |
| Treatment pH | | 8 | 8 | 8 | 8 | 8 | 8 |
| Peptide Ratio | | 0.7 | 1.7 | 1.8 | 3.0 | 3.7 | 0.9 |
| Peptide Ratio Improvement Index | | (Reference) | 2.43 | 2.57 | 4.29 | 5.29 | |

In Table, a numerical value indicating the amount of each enzyme used is represented by an activity value per 1 g of yeast.
† Bacillus stearothermophilus is reclassified as Geobacillus stearothermophilus.

As shown in Tables 2 and 3, the peptide ratio in the obtained yeast extract was found to be remarkably improved when a predetermined enzyme (that is, a predetermined protease selected from the group consisting of a protease derived from the genus Bacillus, a protease derived from the genus Geobacillus, and a protease derived from the genus Rhizopus, and/or a glutaminase) was used (Examples 1 to 4 and 5 to 8) as compared with the case where the yeast extract was produced without using the enzyme (Comparative Examples 1 and 5). In view of the fact that when a predetermined enzyme was not used, the peptide ratio in the obtained yeast extract was hardly improved (Comparative Examples 2 to 4), or the peptide ratio did not exceed 1 in the first place (Comparative Example 6), the remarkable improving effect of the peptide ratio observed in Examples 1 to 4 and 5 to 8 was found to be a specific effect provided by using the predetermined enzyme.

Among Examples 1 to 4 and 5 to 8, when a predetermined protease and glutaminase were combined (Examples 4 and 8), the effect of improving the peptide ratio was especially remarkable.

Furthermore, as shown in comparison between Comparative Example 1 and Comparative Example 5, in view of the fact that the peptide ratio was particularly low when the yeast extract was produced at a high pH (Comparative Example 5), the effect of improving the peptide ratio observed when the yeast extract was produced using a predetermined enzyme at a high pH (Examples 5 to 8) was found to be especially remarkable.

[Test Example 2]

The yeast extract (supernatant) obtained in Test Example 1 was concentrated under reduced pressure (60°C, -1 bar), and then spray-dried (inlet temperature: 140°C, outlet temperature: 100°C) to obtain a dry yeast extract powder.

MRS media having compositions shown in the following Table were prepared. The blending amount of the yeast extract was adjusted so as to be equivalent to a protein amount (the absorbance was confirmed at 280 nm) corresponding to a 5 g/L yeast extract manufactured by Funakoshi Co., Ltd.

[Table 4]

| MRS medium | | |
|---|---|---|
| Proteose peptone | Sigma-Aldrich | 10 g/L |
| Beef extract | Sigma-Aldrich | 10 g/L |
| Yeast Extract | Prepared in Test Example 1 | (*) |
| Glucose | Sigma-Aldrich | 20 g/L |
| Tween80 | Sigma-Aldrich | 1 g/L |
| Ammonium citrate | Sigma-Aldrich | 2 g/L |
| Sodium acetate | Sigma-Aldrich | 5 g/L |
| Magnesium sulphate | Sigma-Aldrich | 0.1 g/L |
| Manganese sulphate | Sigma-Aldrich | 0.005 g/L |
| Dipotassium phosphate | Sigma-Aldrich | 2 g/L |
| (*) Adjusted so as to be equivalent to a protein amount corresponding to 5 g/L of a yeast extract manufactured by Funakoshi Co., Ltd. | | |

The lactic acid bacteria were selected from lactic acid bacteria shown in the following Table. The lactic acid bacteria were inoculated from a glycerol stock into each of the MRS media (the yeast extracts in the MRS media shown in Table 4 were replaced with a 5 g/L yeast extract manufactured by Funakoshi Co., Ltd.), and pre-cultured by static culture at 37°C for 3 days under anaerobic conditions.

[Table 5]

| | ATCC# | Details |
|---|---|---|
| lactic acid bacteria a | 4356 | Lactobacillus acidophilus |
| lactic acid bacteria b | 393 | Lactobacillus casei; Strain3 |
| lactic acid bacteria c | 7469 | Lactobacillus rhamnosus |
| lactic acid bacteria d | 33323 | Lactobacillus gasseri |
| lactic acid bacteria e | 15700 | Bifidobacterium |

[Test Example 2-1]

10 μL of a pre-culture solution was inoculated into 4 mL of each of the MRS media described in Table 4, and static culture was performed at 37°C for 3 days under anaerobic conditions until reaching a stationary phase, thereby performing main culture (n = 7). A dry powder of the yeast extract prepared in Example 4 or a dry powder of the yeast extract prepared in Comparative Example 1 for comparison was used as a yeast extract to be blended in the MRS medium, and a pre-culture solution of each of lactic acid bacteria a, b, c, d, and e was used as the pre-culture solution. Using turbidity (OD660) at the end of culture as an index, a proliferation promoting effect was evaluated by T test. Specifically, the turbidity when the yeast extract of Example 4 was used was converted into a relative value when the turbidity when the yeast extract for comparison was used was defined as 1, and the proliferation promoting effect was evaluated based on the relative value (hereinafter, described as a proliferation promoting index). When the proliferation promoting index exceeds 1, the effect of promoting the production of lactic acid bacteria can be evaluated to be exhibited.

As a result, the yeast extract of Example 4 had a proliferation promoting index of lactic acid bacteria a of 1.05, a proliferation promoting index of lactic acid bacteria b of 1.13, a proliferation promoting index of lactic acid bacteria c of 1.02, a proliferation promoting index of lactic acid bacteria d of 1.12, and a proliferation promoting index of lactic acid bacteria e of 1.02, and was found to have a proliferation promoting effect.

[Test Example 2-2]

A proliferation promoting effect was evaluated in the same manner as in Test Example 2-1, except that the dry powder of the yeast extract prepared in Example 1 was used as a yeast extract to be blended in an MRS medium, and a pre-culture solution of each of lactic acid bacteria b and c was used as a pre-culture solution.

As a result, the yeast extract of Example 1 had a proliferation promoting index of lactic acid bacteria b of 1.11, and a proliferation promoting index of lactic acid bacteria c of 1.07, and was found to have a proliferation promoting effect.

[Test Example 2-3]

A proliferation promoting effect was evaluated in the same manner as in Test Example 2-1, except that the dry powder of the yeast extract prepared in Example 2 was used as a yeast extract to be blended in an MRS medium, and a pre-culture solution of lactic acid bacteria a was used as a pre-culture solution.

As a result, the yeast extract of Example 2 had a proliferation promoting index of lactic acid bacteria a of 1.05, and was found to have a proliferation promoting effect.

[Test Example 2-4]

A proliferation promoting effect was evaluated in the same manner as in Test Example 2-1, except that the dry powder of the yeast extract prepared in each of Examples 6, 7, and 8 or the dry powder of the yeast extract prepared in Comparative Example 5 for comparison was used as a yeast extract to be blended in an MRS medium, and a pre-culture solution of lactic acid bacteria a was used as a pre-culture solution.

As a result, the yeast extract of Example 6 had a proliferation promoting index of lactic acid bacteria a of 1.09, the yeast extract of Example 7 had a proliferation promoting index of lactic acid bacteria a of 1.14, and the yeast extract of Example 8 had a proliferation promoting index of lactic acid bacteria a of 1.08, and the yeast extracts were found to have a proliferation promoting effect.

## Claims

1. A yeast extract production method comprising the step of causing a protease and/or a glutaminase to act on yeast, the protease being selected from the group consisting of a protease derived from the genus Bacillus, a protease derived from the genus Geobacillus, and a protease derived from the genus Rhizopus.

2. The production method according to claim 1, wherein the protease derived from the genus Bacillus is a protease derived from Bacillus licheniformis.

3. The production method according to claim 1, wherein the protease derived from the genus Geobacillus is a protease derived from Geobacillus stearothermophilus.

4. The production method according to claim 1, wherein the protease derived from the genus Rhizopus is a protease derived from Rhizopus niveus.

5. The production method according to claim 1, wherein the step is carried out under the condition of pH 7 to 9 using at least the protease derived from the genus Bacillus and/or the protease derived from the genus Geobacillus.

6. The production method according to claim 1, wherein the step is carried out under the condition of pH 3 to 6 using at least the protease derived from the genus Rhizopus.

7. A yeast extract obtained by the production method according to claim 1.

8. An enhancer for a peptide ratio of a yeast extract, comprising a protease and/or a glutaminase, the protease being selected from the group consisting of a protease derived from the genus Bacillus, a protease derived from the genus Geobacillus, and a protease derived from the genus Rhizopus.

9. An enhancer for an ability to produce lactic acid bacteria of a yeast extract, comprising a protease and/or a glutaminase, the protease being selected from the group consisting of a protease derived from the genus Bacillus, a protease derived from the genus Geobacillus, and a protease derived from the genus Rhizopus.

10. A method for producing a culture of lactic acid bacteria, comprising the step of culturing lactic acid bacteria using a medium containing a yeast extract obtained by the production method according to claim 1.

## INTERNATIONAL SEARCH REPORT

| International application No. |
|---|
| **PCT/JP2023/027110** |

| **A.** | **CLASSIFICATION OF SUBJECT MATTER** |
|---|---|

*A23L 31/15*(2016.01)i; *C12N 1/20*(2006.01)i; *C12N 9/54*(2006.01)i; *C12N 9/56*(2006.01)i; *C12N 9/58*(2006.01)i
FI: A23L31/15; C12N1/20 A; C12N9/54; C12N9/56; C12N9/58

According to International Patent Classification (IPC) or to both national classification and IPC

| **B.** | **FIELDS SEARCHED** |
|---|---|

Minimum documentation searched (classification system followed by classification symbols)

A23L31/15; C12N1/20; C12N9/54; C12N9/56; C12N9/58

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); FSTA/CAplus/AGRICOLA/BIOSIS/MEDLINE/EMBASE (STN)

| **C.** | **DOCUMENTS CONSIDERED TO BE RELEVANT** |
|---|---|

| Category* | Citation of document, with indication, where appropriate, of the relevant passages | Relevant to claim No. |
|---|---|---|
| X | JP 2005-245438 A (NIPPON PAPER CHEMICALS CO., LTD.) 15 September 2005 (2005-09-15) examples 1-3, table 1, claims | 1-5, 7-10 |
| Y | | 1-10 |
| Y | WO 2016/080490 A1 (TABLEMARK CO., LTD.) 26 May 2016 (2016-05-26) paragraphs [0020]-[0023], table 1 | 1-10 |
| X | WO 00/30474 A1 (AJINOMOTO CO., INC.) 02 June 2000 (2000-06-02) examples 2, 6 | 1-4, 7-10 |
| A | | 5-6 |
| A | WO 2021/002195 A1 (AMANO ENZYME INC.) 07 January 2021 (2021-01-07) claims, examples | 1-10 |
| P, A | WO 2022/191303 A1 (AMANO ENZYME U.S.A. CO., LTD.) 15 September 2022 (2022-09-15) claims, examples | 1-10 |

☐ Further documents are listed in the continuation of Box C.　　☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **19 September 2023** | **03 October 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**
Information on patent family members

International application No.

**PCT/JP2023/027110**

| Patent document cited in search report | | | Publication date (day/month/year) | Patent family member(s) | | | Publication date (day/month/year) |
|---|---|---|---|---|---|---|---|
| JP | 2005-245438 | A | 15 September 2005 | (Family: none) | | | |
| WO | 2016/080490 | A1 | 26 May 2016 | US paragraphs [0043]-[0047], table 1 | 2017/0347697 | A1 | |
| | | | | EP | 3222156 | A1 | |
| WO | 00/30474 | A1 | 02 June 2000 | US examples 2, 6 | 2004/0265471 | A1 | |
| | | | | JP | 3733585 | B2 | |
| | | | | EP | 1142493 | A1 | |
| WO | 2021/002195 | A1 | 07 January 2021 | US claims, examples | 2022/0243235 | A1 | |
| | | | | EP | 3995585 | A1 | |
| WO | 2022/191303 | A1 | 15 September 2022 | (Family: none) | | | |

Form PCT/ISA/210 (patent family annex) (January 2015)

## REFERENCES CITED IN THE DESCRIPTION

**Patent documents cited in the description**

- JP 6113789 A

- JP 2009107962 A